(19) Europäisches Patentamt

European Patent Office

Office européen des brevets

(11) **EP 1 245 277 A1**

(12) **EUROPEAN PATENT APPLICATION**

(43) Date of publication:
**02.10.2002 Bulletin 2002/40**

(51) Int Cl.[7]: **B01J 23/30**, B01J 23/18,
B01J 23/20, C07C 45/36,
C07C 47/52

(21) Application number: **02007428.2**

(22) Date of filing: **28.03.2002**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **30.03.2001 JP 2001099128**

(71) Applicant: **Nippon Shokubau Co.,Ltd.
Osaka-shi, Osaka 541-0043 (JP)**

(72) Inventors:
• **Kishimoto, Nobuji
Himeji-shi, Hyogo 670-0114 (JP)**

• **Takahashi, Kunika
Kakogawa-shi, Hyogo 675-0114 (JP)**
• **Nakajima, Akiyoshi
Akashi-shi, Hyogo 674-0092 (JP)**

(74) Representative: **Brehm, Hans-Peter et al
Patent- und Rechtsanwälte
Hansmann & Vogeser
Albert-Rosshaupter-Strasse 65
81369 München (DE)**

(54) **Catalyst for the oxidation of alkylbenzenes to aromatic aldehydes**

(57) The present invention provide a catalyst capable of producing an aromatic aldehyde by gas-phase oxidation of the corresponding alkylbenzene in the presence of a molecular oxygen in high yield, and a method of producing an aromatic aldehyde from the corresponding alkylbenzene in high yield by using the above catalyst.

An alkylbenzene oxidation catalyst for the production of an aromatic aldehyde by gas-phase oxidation of the corresponding alkylbenzene in the presence of a molecular oxygen,

which comprises tungsten and antimony plus an oxide of at least one metal species selected from the group consisting of niobium, tantalum, zirconium, and titanium or a composite oxide of two or more metal species selected from said group.

EP 1 245 277 A1

**Description**

TECHNICAL FIELD

The present invention relates to a catalyst for oxidation of alkylbenzenes and a method of producing aromatic aldehydes by using said catalyst. More particularly, the present invention relates to a catalyst used suitably for the gas-phase oxidation of an alkylbenzene in the presence of molecular oxygen to produce the corresponding aromatic aldehyde in high yield and a method of producing an aromatic aldehyde by the gas-phase oxidation of the corresponding alkylbenzene in the presence of a molecular oxygen in high yield which comprises using said catalyst.

BRIEF DESCRIPTION OF THE PRIOR ART

Aromatic aldehydes have highly reactive aldehyde groups and, among aromatic compounds in general, are broad in the range of utility. Terephthalaldehyde (TPAL) having two aldehyde groups, in particular, is expected to find application in a broad range of uses such as pharmaceuticals, agrochemicals, dyes, liquid crystal polymers, electrically conductive polymers, and heat-resistant plastics, among other uses, and the advent of an uncostly commercial production process has been awaited.

The attempt to produce TPAL by gas-phase oxidation of p-xylene dates back to many years ago. Japanese Koho Publication Sho-47-2086 discloses a catalyst comprising an oxide in a W to Mo ratio of 1:1 through 20:1 as supported on alumina. Japanese Kokai Publication Sho-48-97830 discloses a catalyst comprising V and Rb or Cs as supported on silicon carbide. USP 3,845,137 discloses a catalyst comprising an oxide composed of the two elements of W and Mo plus at least one element selected from the group consisting of Ca, Ba, Ti, Zr, Hf, Tl, Nb, Zn and Sn as supported on alumina. USP 4,017,547 discloses a catalyst comprising a component which is composed of Mo oxide, W oxide or silicotungstic acid, and Bi oxide all as supported on silicon carbide. USP 5,324,702 discloses a special catalyst comprising Fe, Zn, etc. and V, Mo, W, etc. as supported by chemical vapor deposition (CVD) on deboronized borosilicate crystal molecular sieves.

However, these catalysts are deficient in the yield and productivity of the objective compound TPAL and, therefore, have not been commercially implemented.

The inventors of the present invention also proposed a catalyst comprising oxides of W, S, Fe, etc. (JP Kokai 2001-198464) but its performance was not enough to be commercially implemented.

SUMMARY OF THE INVENTION

The object of the present invention is to provide a novel catalyst capable of producing an aromatic aldehyde by gas-phase oxidation of the corresponding alkylbenzene in the presence of a molecular oxygen and a method of producing an aromatic aldehyde from the corresponding alkylbenzene in high yield by using the above catalyst.

The inventors of the present invention conducted an intensive investigation for finding a novel catalyst capable of producing an aromatic aldehyde by gas-phase oxidation of the corresponding alkylbenzene in the presence of a molecular oxygen in high yield and found that the catalyst described hereinafter exhibits a satisfactory partial oxidation performance and that by using this catalyst, an aromatic aldehyde can be produced in high yield with high productivity.

The present invention, therefore, is directed to a catalyst for the production of an aromatic aldehyde by gas-phase oxidation of the corresponding alkylbenzene in the presence of a molecular oxygen,

which comprises tungsten and antimony plus an oxide of at least one metal species selected from the group consisting of niobium, tantalum, zirconium and titanium or a composite oxide of two or more metal species selected from said group.

The present invention is further directed to a method of producing an aromatic aldehyde by gas-phase oxidation of the corresponding alkylbenzene in the presence of a molecular oxygen,

which comprises using the above catalyst.

EMBODIMENTS OF THE INVENTION

Alkylbenzenes of the present invention are compounds such that one or more alkyl groups are bound directly to a benzene ring and includes, as representative examples, such methylbenzenes as toluene, p-xylene, o-xylene, m-xylene, pseudocumene, mesitylene, durene, etc., ethylbenzene, cumene, p-cymene, p-tert-butyltoluene, and p-diisopropylbenzene, among others.

The catalyst of the invention functions for the production of the corresponding aromatic aldehyde by the gas-phase oxidation of these alkylbenzenes. Specifically, it catalyzes the oxidation of toluene to benzaldehyde; p-xylene to terephthaladehyde and p-tolualdehyde; o-xylene to phthalaldehyde and o-tolualdehyde; m-xylene to isophthalaldehyde, m-tolualdehyde; pseudocumene to 2-methylterephthalaldehyde, 2,4-dimethylbenzaldehyde, 2/5-dimethylbenzaldehyde, and 3,4-dimethylbenzaldehyde; mesitylene to 3,5-dimethylbenzaldehyde, 5-methylisophthalaldehyde and 1,3,5-triformylbenzene; durene to 2,5-dimethylterephthalaldehyde, 4,5-dimethylphthalaldehyde, 2,4,5-trimethylbenzaldehyde, 2,4,5-triformyltoluene and 1,2,4,5-tetraformylbenzene; ethylbenzene or cumene to benzaldehyde; p-cymene to terephthalaldehyde, cuminaldehyde and p-tolualdehyde; p-tert-butyltoluene to p-tert-butylbenzaldehyde; p-diisopropylbenzene to terephthalaldehyde and cuminaldehyde; and so forth. In particular, the oxidation catalyst of the invention can be used suitably for the production of aromatic aldehydes from the corresponding methylbenzenes having 8 to 10 carbon atoms, most preferably for the production of terephthalaldehyde from p-xylene.

The oxidation catalyst of the invention comprises tungsten and antimony plus an oxide of at least one metal species selected from the group consisting of niobium, tantalum, zirconium and titanium or a composite oxide of two or more metal species selected from said group. The preferred catalyst of the invention further contains at least one metal species selected from the group consisting of Zn, Co, Ni, Mn, Mg, Ca, Sr, Li, Na, K, Rb and Cs. Among these metal species, Zn is particularly preferred.

When $\alpha$-alumina, silicon carbide or the like is used in combination with a catalyst component containing tungsten and antimony as proposed in the prior art for the production of aromatic aldehydes by gas-phase oxidation of the corresponding alkylbenzenes, the catalyst activity attainable is considerably low as compared with the case in which the catalyst of the present invention is used. Moreover, in the case of silica or active alumina, which is commonly used as the support, the reaction selectivity is remarkably low. Further, when the character of the invention, namely, the oxide of at least one metal species selected from the group consisting of niobium, tantalum, zirconium and titanium or a composite oxide of two or more metal species selected from said group is not used, the resulting catalyst is not only inadequate in physical durability but also insufficient in both activity and selectivity. In contrast, when said oxide of at least one metal species selected from the group consisting of niobium, tantalum, zirconium and titanium or said composite oxide of two or more metal species selected from the same group is employed as proposed by the present invention, the resulting catalyst is high in both activity and selectivity. Among such oxides, the oxide of titanium is preferred and especially a rutile type titanium oxide can be used with the greatest advantage.

For the production of the catalyst according to the present invention, the form of said oxide or composite oxide of niobium, tantalum, zirconium, or/and titanium is not particularly restricted. For example, it may be a molding or a bulk powder. Moreover, it can also be used in the form of a salt such as hydroxide, which is a precursor of said metal oxide or composite oxide, or in the form of a gel or a sol in the production of the catalyst.

The BET specific surface area of the metal oxide or composite oxide of niobium, tantalum, zirconium or/and titanium used in the present invention is not particularly restricted but, for suppression of side reactions and attainment of the objective product in high yield, is preferably not more than 30 $m^2/g$, especially not more than 10 $m^2/g$. When a precursor is used, too, the baking conditions should be so selected that the specific surface area after conversion to the oxide will be 30 $m^2/g$ or less, preferably 10 $m^2/g$ or less. With the total mass of the catalyst being taken as 100, the amount of the oxide or composite oxide of Nb, Ta, Zr or/and Ti is generally 10 to 95 on a mass basis. The shape of the catalyst is not particularly restricted but may for example be granular, spherical, pellet-like, ring-shaped or honeycomb-shaped depending on the size and geometry of the tubular reactor to be used.

The technology of preparing the oxidation catalyst according to the invention is not particularly restricted but may be any of the methods used generally for the production of catalysts of this type. A typical method may comprise adding an aqueous solution of antimony tartrate or an antimony trioxide powder to an aqueous solution of ammonium metatungstate to give a homogeneous solution or suspension, immersing a molding of said metal oxide in said solution or suspension, and subjecting it to evaporation to dryness or blending said solution or suspension with a powder of said metal oxide or said sol or the like and subjecting the mixture to heating and stirring, concentration, and evaporation to dryness. In either case, the product is dried at 80 to 230°C and, where necessary, crushed or molded, and after size adjustment, baked at 300 to 700°C. The atmosphere in which said drying and baking are carried out is not particularly restricted. Thus, these operations can be carried out in any of atmospheric air, a high-oxygen atmosphere, a low-oxygen atmosphere, a reducing gas atmosphere, or an inert gas, such as nitrogen, helium, argon or the like, or even in vacuo. The above methods are selectively employed according to characteristics of the raw materials used for preparation of the catalyst.

The raw materials which can be used for preparation of the catalyst mentioned above are not particularly restricted. In the case of tungsten, an aqueous solution of ammonium metatungstate, ammonium paratungstate, tungsten trioxide, etc. can be used suitably, and in the case of antimony, antimony trioxide, antimonic acid sol, an aqueous solution of antimony tartrate, etc. can be used suitably. Among these, the preferred form of tungsten is an aqueous solution of ammonium metatungstate and the preferred form of antimony is antimony trioxide or an aqueous solution of antimony tartrate.

The proportion of tungsten in the catalyst, with the total mass of the catalyst being taken as 100, is preferably not less than 10 and not more than 60 on a mass basis, more preferably not less than 15 and not more than 40 on a mass basis. The proportion of said antimony in the catalyst, with the total mass of the catalyst being taken as 100, is preferably not less than 1 and not more than 20, more preferably not less than 2 and not more than 10.

With regard to any optional element that may be added as a catalyst component, too, it may be any of such forms as the nitrate, sulfate, oxide, hydroxide, chloride, carbonate, organic acid salt, metal oxygen acids, metal oxygen acid ammonium salt, heteropolyacid and so on.

The proportion of the optional element that may be added, namely at least one metal species selected from the group consisting of Zn, Co, Ni, Mn, Mg, Ca, Sr, Li, Na, K, Rb and Cs, in the catalyst with the total mass of the catalyst being taken as 100, is preferably not less than 0 and not more than 10, more preferably not less than 0.1 and not more than 5.

The raw materials for the gas-phase oxidation reaction according to the invention include alkylbenzenes and molecular oxygen, and optionally a diluent gas. As the source of molecular oxygen, either air or pure oxygen is used. The molecular oxygen is generally used in a ratio of 5 to 100 moles to each mol of the alkylbenzenes. As the diluent gas, an inert gas, such as nitrogen, helium or carbon dioxide gas, or water vapor can be used suitably.

The reaction conditions for the gas-phase oxidation reaction according to the invention are not particularly restricted. For example, the above material gas can be caused to contact the oxidation catalyst of the invention at a space velocity of 1,000 to 200,000 $h^{-1}$ and a reaction temperature of 350 to 650°C. The preferred space velocity is 10,000 to 100,000 $h^{-1}$ and the preferred reaction temperature is 450 to 600°C. The above reaction is generally carried out at ordinary pressure or slightly elevated pressure but may be conducted optionally at high pressure or at reduced pressure. The reaction mode is not particularly restricted but may be whichever of stationarybed, moving-bed, and fluidized-bed modes. It may also be a one-pass system or a recycling system.

While the alkylbenzene oxidation catalyst of the present invention capable of producing an aromatic aldehyde by gas-phase oxidation of the corresponding alkylbenzene in the presence of a molecular oxygen in high yield, the preferred catalyst is such that when used in the reaction carried out at a space velocity of 10,000-100,000 $h^{-1}$ and a reaction temperature of 520 to 580°C, the yield of the aromatic aldehyde will be at least 50 mol %. If it is less than 50 mol %, useful aromatic aldehydes such as terephthalaldehyde (TPAL) may not be produced economically on a commercial scale. It is more preferably not less than 60 mol %, still more preferably not less than 65 mol %, most preferably not less than 70 mol %.

While the alkylbenzene oxidation catalyst of the present invention is used for producing an aromatic aldehyde by gas-phase oxidation of the corresponding alkylbenzene in the presence of a molecular oxygen, a method of producing an aromatic aldehyde by using such a catalyst is also an aspect of the invention.

The alkylbenzene oxidation catalyst of the present invention exhibits a satisfactory partial oxidation performance and is capable of producing an aromatic aldehyde from the corresponding alkylbenzene in high yield.

EXAMPLES

The following examples illustrate the present invention in further detail. It should be understood that the conversion rate, selectivity, and one-pass yield of the reaction are as defined below.

$$\text{Conversion (mole \%)} = (\text{the number of moles of reacted}$$

$$\text{material/the number of moles of fed material)} \times 100$$

$$\text{Selectivity (mol \%)} = (\text{the number of moles of each}$$

$$\text{compound produced/the number of moles of reacted material)} \times 100$$

$$\text{One-pass yield (mol \%)} = (\text{the number of moles of each}$$

$$\text{compound produced/the number of moles of fed material)} \times 100$$

Example 1

For use as an antimony component, an aqueous solution of antimony tartrate was prepared in the first place. In 310 ml of water was dissolved 150.0 g of L-tartaric acid, and 36.5 g of antimony trioxide was then added and dissolved by refluxing. A small quantity of water was added to this solution to make a total of 500 g. The aqueous solution of antimony tartrate thus obtained had an Sb concentration of 0.5 mmol/g. To 11.25 g of this aqueous solution of antimony tartrate was added 10.42 g of an aqueous solution of ammonium metatungstate (50 mass % as $WO_3$) to prepare a homogeneous impregnating solution. Then, 10.0 g of niobium oxide powder (BET specific surface area 4.9 $m^2$/g) was added to the above impregnating liquor and the mixture was heated with stirring for about 2 hours to effect evaporation to dryness. The residue was dried by heating at 260°C for 16 hours in a stream of $N_2$, then crushed, classified to 16 to 30 mesh size, and baked in an atmosphere at 580°C for 2 hours. The composition of the catalyst thus obtained was 39 mass % $W_1Sb_{0.25}O_x/Nb_2O_5$.

An ordinary flow reactor tube was packed with 0.5 g of the above catalyst and the reaction was carried out under the following conditions. The space velocity (SV) and the results of this reaction are shown in Table 1.

Reaction pressure: atmospheric
Reactant gas composition: p-xylene/air = 0.8/99.2
        (p-xylene/$O_2$ = 1/25.8)
Reactant gas feeding rate: 180 ml (standard state)/min.
Reaction temperature: 560°C

Examples 2 to 4

Using 10.0 g of each tantalum oxide powder (BET specific surface area 2.5 $m^2$/g), zirconium oxide powder (BET specific surface area 8.8 $m^2$/g), or titanium oxide powder (anatase type, BET specific surface area 20.0 $m^2$/g) in lieu of niobium oxide, the procedure of Example 1 was otherwise repeated to prepare catalysts.

Using 0.2 g (Examples 2 and 3) or 0.3 g (Example 4) each of these catalysts, the reaction was carried out under otherwise the same conditions as in Example 1. The SV and results of the reaction are shown in Table 1.

Comparative Example 1

Except that niobium oxide was not used, the procedure of Example 1 was otherwise repeated to prepare a catalyst. The resulting catalyst composition was $W_1Sb_{0.25}O_x$. Using 0.5 g of this catalyst, the reaction was carried out under otherwise the same conditions as in Example 1. The SV and results of the reaction are shown in Table 1.

Comparative Examples 2 to 5

Using 10.0 g aluminum oxide powder (BET specific surface area 0.8 $m^2$/g), 48.8 g of silica sol (Nissan Chemical's Snowtex-O, 20.5 mass % as $SiO_2$), 10.0 g of zinc oxide powder (BET specific surface area 3.9 $m^2$/g), and 10.0 g of tin oxide (BET specific surface area 2.5 $m^2$/g), respectively, the procedure of Example 1 was otherwise repeated to prepare catalysts.

Using 0.5 g each of the above catalysts, the reaction was respectively carried out under the same conditions as in Example 1. The SV values and results of the reaction are presented in Table 1.

Table 1

| | Support | SV ($h^{-1}$) | PX conversion (mole %) | Selectivity (mole %) | | One-pass yield (mole %) | |
|---|---|---|---|---|---|---|---|
| | | | | TPAL | PTAL | TPAL | PTAL |
| Ex. 1 | $Nb_2O_5$ | 28000 | 90.9 | 69.4 | 4.3 | 63.1 | 3.9 |
| Ex. 2 | $Ta_2O_5$ | 95000 | 90.8 | 64.9 | 5.8 | 58.9 | 5.3 |
| Ex. 3 | $ZrO_2$ | 70000 | 91.6 | 62.9 | 5.4 | 57.6 | 4.9 |
| Ex.4 | $TiO_2$ | 40000 | 95.9 | 63.8 | 3.5 | 61.2 | 3.4 |
| Compar. Ex. 1 | - | 38000 | 62.8 | 54.2 | 4.7 | 34.0 | 3.0 |
| Compar. Ex. 2 | $Al_2O_3$ | 22000 | 42.2 | 60.1 | 5.2 | 25.4 | 2.2 |
| Compar. Ex. 3 | $SiO_2$ | 15000 | 95.9 | 10.5 | 2.8 | 10.1 | 2.7 |
| Compar. Ex. 4 | ZnO | 23500 | 21.7 | 3.6 | 4.2 | 0.8 | 0.9 |
| Compar. Ex. 5 | SnO | 28000 | 23.4 | 6.7 | 4.1 | 1.6 | 1.0 |
| PX:p-Xylene, TPAL:Terephthalaldehyde, PTAL:p-Tolualdehyde | | | | | | | |

Example 5

Except that the baking temperature was changed to 640°C, the procedure of Example 4 was otherwise repeated to prepare a catalyst. Using 1.0 g of this catalyst and a reaction temperature of 550°C, the reaction was carried out under otherwise the same conditions as in Example 1. The SV was 12,000 h$^{-1}$. The results of the reaction were: conversion of p-xylene = 90.2 mol %, selectivity for terephthalaldehyde = 68.7 mol %, selectivity for tolualdehyde = 5.1 mol %, one-pass yield of terephthalaldehyde = 62.0 mol %, and one-pass yield of tolualdehyde = 4.6 mol %.

Comparative Example 6

Except that Sb was not used, the procedure of Example 5 was otherwise repeated to prepare a catalyst. Then, using 1.0 g of this catalyst, the reaction was carried out under the same conditions as in Example 5. The results of the reaction were: conversion of p-xylene = 100 mol %; neither terephalaldehyde nor tolualdehyde produced, only CO and $CO_2$ were produced.

Example 6

Except that an anatase titanium oxide powder was changed to a rutile one (BET specific surface area 1.0 m$^2$/g), the procedure of Example 5 was otherwise repeated to prepare a catalyst. Using 1.0 g of this catalyst and a reaction temperature of 570°C, the reaction was carried out under otherwise the same conditions as in Example 5. The SV was 10,500 h$^{-1}$. The results of the reaction are presented in Table 2.

Examples 7 to 11

Except that an impregnating solution was prepared by adding an aqueous solution of zinc nitrate (Example 7), an aqueous solution of cobalt nitrate (Example 8), an aqueous solution of nickel nitrate (Example 9), an aqueous solution of calcium nitrate (Example 10), or an aqueous solution of potassium nitrate (Example 11), respectively, to the aqueous solution of antimony tartrate and aqueous solution of ammonium metatungstate used in Example 6, the procedure of Example 6 was otherwise repeated to prepare catalysts. Using 1.0 g of each catalyst thus prepared, the reaction was carried out under otherwise the same conditions as in Example 6. The formulations of metals added to $W_1Sb_{0.25}$ and the results of the reaction are presented in Table 2.

Table 2

|  | Catalyst formulation | PX conversion (mole %) | Selectivity (mole %) | | One-pass yield (mole%) | |
|---|---|---|---|---|---|---|
|  |  |  | TPAL | PTAL | TPAL | PTAL |
| Ex.6 | - | 98.5 | 64.2 | 4.6 | 63.2 | 4.5 |
| Ex.7 | Zn 0.04 | 97.5 | 72.0 | 5.0 | 70.2 | 4.9 |
| Ex.8 | Co 0.04 | 96.2 | 68.9 | 4.5 | 66.3 | 4.3 |
| Ex.9 | Ni 0.04 | 97.6 | 66.8 | 4.5 | 65.2 | 4.4 |
| Ex. 10 | Ca 0.04 | 97.1 | 67.1 | 4.7 | 65.2 | 4.6 |
| Ex. 11 | K 0.03 | 86.6 | 75.8 | 4.2 | 65.6 | 3.6 |
| PX:p-Xylene, TPAL:Terephthalaldehyde, PTAL:p-Tolualdehyde | | | | | | |

**Claims**

1. An alkylbenzene oxidation catalyst for the production of an aromatic aldehyde by gas-phase oxidation of the corresponding alkylbenzene in the presence of a molecular oxygen,
   which comprises tungsten and antimony plus an oxide of at least one metal species selected from the group consisting of niobium, tantalum, zirconium, and titanium or a composite oxide of two or more metal species selected from said group.

2. A method of producing an aromatic aldehyde by the gas-phase oxidation of the corresponding alkylbenzene in the presence of a molecular oxygen,

which comprises using the catalyst according to Claim 1.

**European Patent Office**

**EUROPEAN SEARCH REPORT**

Application Number

EP 02 00 7428

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,X | EP 1 099 475 A (NIPPON CATALYTIC CHEM IND) 16 May 2001 (2001-05-16) * paragraph '0001! * * paragraph '0021! * * paragraph '0022! * * page 4, line 9 - line 12 * * examples 12,25 * | 1,2 | B01J23/30 B01J23/18 B01J23/20 C07C45/36 C07C47/52 |
| X | EP 0 958 860 A (NIPPON CATALYTIC CHEM IND) 24 November 1999 (1999-11-24) * paragraph '0009! * * paragraph '0017! * | 1 | |
| X | EP 0 295 768 A (STANDARD OIL CO OHIO) 21 December 1988 (1988-12-21) * page 3, column 3, line 1 - line 17 * * page 4, column 6, line 32 - line 42 * * example 7 * | 1 | |
| X | US 3 923 819 A (LUSSLING THEODOR ET AL) 2 December 1975 (1975-12-02) * example 17 * * column 2, line 16 - line 21 * * column 3, line 6 - line 9 * | 1 | TECHNICAL FIELDS SEARCHED (Int.Cl.7) B01J C07C |
| X | US 5 324 702 A (YOO JIN S ET AL) 28 June 1994 (1994-06-28) * column 2, line 15 - line 22 * * column 5, line 47 - column 6, line 6 * * column 2, line 44 - line 64 * | 1,2 | |
| X | GB 2 144 049 A (NITTO CHEMICAL INDUSTRY CO LTD) 27 February 1985 (1985-02-27) * page 3, line 2 - line 31 * * page 3, line 36 - line 40 * * page 6, line 43 - line 52 * | 1,2 | |

The present search report has been drawn up for all claims

| Place of search | Date of completion of the search | Examiner |
|---|---|---|
| MUNICH | 4 July 2002 | Holzwarth, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                     EP 02 00 7428

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2002

| Patent document cited in search report | | | Publication date | Patent family member(s) | | Publication date |
|---|---|---|---|---|---|---|
| EP 1099475 | A | | 16-05-2001 | EP | 1099475 A1 | 16-05-2001 |
| | | | | JP | 2001198464 A | 24-07-2001 |
| EP 0958860 | A | | 24-11-1999 | EP | 0958860 A2 | 24-11-1999 |
| | | | | JP | 2000037624 A | 08-02-2000 |
| | | | | SG | 80627 A1 | 22-05-2001 |
| | | | | US | 6239325 B1 | 29-05-2001 |
| EP 0295768 | A | | 21-12-1988 | US | 4837233 A | 06-06-1989 |
| | | | | AT | 98221 T | 15-12-1993 |
| | | | | BR | 8801841 A | 22-11-1988 |
| | | | | CA | 1305121 A1 | 14-07-1992 |
| | | | | CN | 88102076 A ,B | 30-11-1988 |
| | | | | CN | 1062306 A | 01-07-1992 |
| | | | | DE | 3886103 D1 | 20-01-1994 |
| | | | | DE | 3886103 T2 | 14-04-1994 |
| | | | | EP | 0295768 A2 | 21-12-1988 |
| | | | | ES | 2059511 T3 | 16-11-1994 |
| | | | | JP | 1038051 A | 08-02-1989 |
| | | | | MX | 167184 B | 09-03-1993 |
| | | | | TR | 23989 A | 14-01-1991 |
| US 3923819 | A | | 02-12-1975 | DE | 1948715 A1 | 01-04-1971 |
| | | | | AT | 549874 A | 15-04-1976 |
| | | | | AT | 332856 B | 25-10-1976 |
| | | | | AT | 868970 A | 15-02-1976 |
| | | | | BE | 756614 A1 | 01-03-1971 |
| | | | | CA | 961835 A1 | 28-01-1975 |
| | | | | CH | 549411 A | 31-05-1974 |
| | | | | FR | 2062524 A5 | 25-06-1971 |
| | | | | GB | 1332831 A | 03-10-1973 |
| | | | | GB | 1332832 A | 03-10-1973 |
| | | | | NL | 7011840 A ,B | 30-03-1971 |
| | | | | SE | 383876 B | 05-04-1976 |
| | | | | SE | 7315249 A | 09-11-1973 |
| | | | | US | 3926856 A | 16-12-1975 |
| US 5324702 | A | | 28-06-1994 | NONE | | |
| GB 2144049 | A | | 27-02-1985 | JP | 1618310 C | 12-09-1991 |
| | | | | JP | 2045500 B | 09-10-1990 |
| | | | | JP | 58139745 A | 19-08-1983 |
| | | | | AT | 384558 B | 10-12-1987 |
| | | | | AT | 189882 A | 15-05-1987 |
| | | | | BG | 50715 A3 | 15-10-1992 |
| | | | | BR | 8202794 A | 26-04-1983 |

EPO FORM P0459

For more details about this annex : see Official Journal of the European Patent Office, No. 12/82

EP 1 245 277 A1

## ANNEX TO THE EUROPEAN SEARCH REPORT
## ON EUROPEAN PATENT APPLICATION NO.

EP 02 00 7428

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2002

| Patent document cited in search report | Publication date | Patent family member(s) | | Publication date |
|---|---|---|---|---|
| GB 2144049 A | | CA | 1189844 A1 | 02-07-1985 |
| | | DD | 202630 A5 | 28-09-1983 |
| | | DD | 210218 A5 | 06-06-1984 |
| | | DE | 3217700 A1 | 02-12-1982 |
| | | ES | 512235 D0 | 01-06-1983 |
| | | ES | 8306607 A1 | 16-09-1983 |
| | | ES | 519785 D0 | 01-03-1984 |
| | | ES | 8403040 A1 | 01-06-1984 |
| | | FR | 2505675 A1 | 19-11-1982 |
| | | GB | 2101004 A ,B | 12-01-1983 |
| | | GB | 2163365 A ,B | 26-02-1986 |
| | | IN | 162232 A1 | 16-04-1988 |
| | | IT | 1154303 B | 21-01-1987 |
| | | KR | 8903702 B1 | 30-09-1989 |
| | | MX | 162726 A | 20-06-1991 |
| | | NL | 8202012 A | 01-12-1982 |
| | | SU | 1367844 A3 | 15-01-1988 |
| | | US | 4618593 A | 21-10-1986 |
| | | US | 4709070 A | 24-11-1987 |
| | | US | 4709071 A | 24-11-1987 |

EPO FORM P0459

For more details about this annex : see Official Journal of the European Patent Office, No. 12/82

10